# EUROPÄISCHE PATENTSCHRIFT

(11) **EP 3 787 722 B1**
(45) Veröffentlichungstag und Bekanntmachung des Hinweises auf die Patenterteilung: **19.07.2023**
(21) Anmeldenummer: 19720566.9
(22) Anmeldetag: 29.04.2019
(51) Int. Cl.: A61M 16/04

(54) **TRACHEALBEATMUNGSVORRICHTUNG MIT EINER ABDICHTUNG**
TRACHEAL RESPIRATORY DEVICE HAVING A SEALING
DISPOSITIF DE RESPIRATION TRACHÉALE DOTÉ D'UNE ÉTANCHÉITÉ

(30) Priorität: 30.04.2018 DE 102018110352
(43) Veröffentlichungstag der Anmeldung: 10.03.2021
(73) Patentinhaber: Coloplast A/S, 3050 Humlebaek (DK)
(72) Erfinder: SCHNELL, Ralf, 63500 Seligenstadt (DE)
(86) Internationale Anmeldenummer: PCT/EP2019/060860
(87) Internationale Veröffentlichungsnummer: WO 2019/211216

(56) Entgegenhaltungen:
- EP-A1- 1 803 478
- DE-U1-202006 006 786
- US-A1- 2009 235 936
- US-A1- 2015 122 264

## Beschreibung

Die vorliegende Erfindung betrifft eine Trachealbeatmungsvorrichtung, insbesondere Tracheostomiekanüle, mit einer Außenkanüle und einer Innenkanüle, wobei der Innenquerschnitt der Außenkanüle und der Außenquerschnitt der Innenkanüle derart aufeinander abgestimmt sind, dass die Innenkanüle mit Spiel in der Außenkanüle bewegbar ist.

Trachealbeatmungsvorrichtungen werden in die Trachea eines Patienten eingeführt, damit der Patient entweder aktiv und/oder passiv ein- und ausatmen kann. Das proximale Ende der Trachealbeatmungsvorrichtung ist außerhalb des Patienten mit einer Beatmungsvorrichtung verbindbar, während das distale Ende der Trachealbeatmungsvorrichtung in die Trachea einführbar ist. Die Trachealbeatmungsvorrichtung dient der Hindurchleitung des Atemgases (Atemluft). Sie wird durch eine künstlich erzeugte Öffnung in die Trachea eines Patienten eingeführt.

Die Begriffe "distal" und "proximal" werden im Sinne der vorliegenden Erfindung aus der Sicht eines behandelnden Arztes verwendet, d.h. dass das proximale Ende der Trachealbeatmungsvorrichtung bzw. der Außenkanüle außerhalb des Patientenkörpers liegt, während das distale Ende im eingebrachten Zustand der Trachealbeatmungsvorrichtung im Inneren der Trachea liegt.

Der Atemwiderstand ist bei einer Atmung durch eine Trachealbeatmungsvorrichtung geringer als der Atemwiderstand bei einer Atmung über die natürlichen Atemwege. Um nach einer längeren Phase der Atmung mit einer Trachealbeatmungsvorrichtung den Patienten an den höheren Atemwiderstand der natürlichen Atemwege zu gewöhnen, wird häufig die proximale Öffnung der Außenkanüle zeitweise ganz oder teilweise verschlossen. Der Patient wird auf diese Weise gezwungen die oberen natürlichen Atemwege zu verwenden und die Atemluft durch einen Luftspalt zwischen der in die Trachea eingeführten Außenkanüle und der Trachealwand zu führen. Durch den kleinen Luftspalt ist der Atemwiderstand für einen Patienten jedoch meist ungewohnt hoch und ermüdend. Häufig reicht die durchgeleitete Atemmenge nicht aus, um den Patienten eine klar verständliche Kommunikation zu ermöglichen.

Mit Hilfe einer Phonationsöffnung in der Tracheostomiekanüle kann der Atemwiderstand reduziert werden. Die Phonationsöffnung ist eine in dem Kanülenrohr zusätzlich zu den zwei proximalen und distalen Öffnungen angeordnete, dritte Öffnung. Durch die lateral angeordnete Phonationsöffnung kann Atemluft aus der Tracheostomiekanüle über die oberen Atemwege geführt werden. Auf diese Weise trägt die Phonationsöffnung erheblich zur Verbesserung des Sprechvermögens bei. Dank einer lauteren und deutlicheren Aussprache kann der Patient besser kommunizieren.

Für eine künstliche Beatmung ist die Verwendung einer Tracheostomiekanüle mit Phonationsöffnung dagegen aus verschiedenen Gründen ungeeignet. Beispielsweise würde die Phonationsöffnung bewirken, dass Beatmungsluft undefiniert durch die oberen Atemwege nach außen strömen kann, so dass möglicherweise nicht mehr genügend Sauerstoff in die Lunge gelangt. Da Beatmungsgeräte die Beatmung mittels Drucksensoren überwachen, würde ein Druckabfall aufgrund der undefiniert durch die Phonationsöffnung abströmenden Atemluft eine Leckage erkennen und einen Alarm ausgeben. Durch eine permanent geöffnete Phonationsöffnung kann zudem Sekret aus dem subglottischen Bereich in die Außenkanüle und durch die distale Öffnung der Außenkanüle in die unteren Atemwege gelangen und Entzündungen verursachen. Bei einem notwendigen Wechsel auf künstliche Beatmung müsste demzufolge eine zuvor verwendete Kanüle mit Phonationsöffnung ausgetauscht werden.

Jeder Wechsel der Trachealbeatmungsvorrichtung ist für den Patienten unangenehm und auch mit Risiken verbunden. Ein wiederholtes Auswechseln einer Trachealbeatmungsvorrichtung ist daher unerwünscht, andererseits aber auch aus hygienischen oder therapeutischen Gründen oft unvermeidliche. Um den Wechsel von Tracheostomiekanülen zu erleichtern, stehen bereits Trachealbeatmungsvorrichtungen mit Außen-und Innenkanüle zur Verfügung, bei welchen in vielen Fällen lediglich die nicht in unmittelbaren Körperkontakt gelangenden Innenkanülen getauscht werden müssen. Die Innenkanüle wird dabei durch die proximale Öffnung der Außenkanüle eingeführt. Der Innenquerschnitt der Außenkanüle und der Außenquerschnitt der Innenkanüle sind derart aufeinander abgestimmt, dass die Innenkanüle mit etwas Spiel in die Außenkanüle eingeführt werden kann. Durch Entfernen der Innenkanüle aus der Außenkanüle kann die Phonationsöffnung genutzt werden. Alternativ könnte auch die Innenkanüle eine Phonationsöffnung aufweisen, die mit der Phonationsöffnung der Außenkanüle überlappt. Ideal ist eine Innenkanüle, die durch Drehung um die Längsachse der Kanüle wahlweise zur Überdeckung mit der Phonationsöffnung der Außenkanüle und ansonsten einem geschlossenen Wandabschnitt der Außenkanüle gegenüber liegt.

Unabhängig davon, ob die Außenkanüle eine Phonationsöffnung aufweist oder nicht, ist es häufig aus hygienischen Gründen sowie aus Sicherheitsaspekten wünschenswert, eine Innenkanüle in die Außenkanüle einer herkömmlichen Trachealbeatmungsvorrichtung einzuführen bzw. eine Innenkanüle auszutauschen.

Damit die Innenkanüle ohne unnötig hohen Kraftaufwand in die Außenkanüle geschoben werden kann, gibt es zwischen der Außenkanüle und der in die Außenkanüle eingeführten Innenkanüle ein ausreichendes Spiel bzw. einen Spalt. Die Größe des Spiels ist so dimensioniert, dass die Einführung der Innenkanüle in die Außenkanüle trotz Fertigungstoleranzen gewährleistet ist.

Allerdings kann durch den Spalt auch Atemgas gelangen. Liegt die Phonationsöffnung versehentlich im Stomakanal, kann die durch den Spalt austretende Luft im ungünstigen Fall zu einem Emphysem führen. Auch kann in den oberen Atemwegen gebildetes Sekret ungewollt durch den Spalt hindurchtreten und so in die Bronchien und die Lunge des Patienten gelangen und möglicherweise zu einer Pneumonie führen. Aus der US 2015/122264 A1 ist eine Trachealbeatmungsvorrichtung nach dem Oberbegriff des Anspruchs 1 bekannt.

Vor diesem Hintergrund ist es eine Aufgabe der vorliegenden Erfindung eine herkömmliche Trachealbeatmungsvorrichtung mit Außen- und Innenkanüle bereitzustellen, bei der die Innenkanüle in der Außenkanüle wirksam abdichtbar ist, ohne das Ein- und Ausführen der Innenkanüle in die Außenkanüle wesentlich zu erschweren.

Erfindungsgemäß wird die Aufgabe durch eine Trachealbeatmungsvorrichtung der eingangs genannten Art gelöst, wobei ein radial verformbarer Schlauchabschnitt vorgesehen ist, dessen Enden mit der Außenkanüle dicht verbunden sind, wobei ein Bereich des Schlauchabschnittes zwischen dessen Enden aus einem ersten Zustand in einen zweiten Zustand radial bewegbar ist, und umgekehrt, wobei in dem ersten Zustand ein Spiel auch in dem Bereich des radial bewegbaren Schlauchabschnitts zwischen Außen- und Innenkanüle vorliegt und in dem zweiten Zustand der Schlauchabschnitt die in die Außenkanüle eingesetzte Innenkanüle gegenüber der Außenkanüle abdichtet.

Zum Ein- und Ausbringen der Innenkanüle wird der Schlauchabschnitt in den ersten Zustand gebracht, so dass die Innenkanüle problemlos axial in die Außenkanüle hinein und aus dieser heraus bewegbar ist, während nach dem Einbringen der Innenkanüle der erwähnte Schlauchabschnitt in den zweiten Zustand gebracht wird und dann den Spalt zwischen Außen- und Innenkanüle entlang des Umfangs der Innenkanüle entlang einer axialen Position abdichtet, die distal zu einer etwaigen Phonationsöffnung und auf gleicher axialer Höhe im Vergleich zu einem gegebenenfalls vorhandenen Cuff liegt. Der Schlauchabschnitt trägt dann auch zu einer axialen Fixierung der Innenkanüle in der Außenkanüle bei.

Sind die Enden des radial verformbaren Schlauchabschnittes mit der Außenkanüle dicht verbunden, kann der Schlauchabschnitt zwischen diesen Enden zur Abdichtung einer in die Außenkanüle eingesetzten Innenkanüle radial in Richtung der Innenkanüle verjüngt werden. Der Schlauchabschnitt schmiegt sich an die Außenfläche der Innenkanüle an und dichtet so einen Spalt zwischen der Außen- und Innenkanüle ab. Im Sinne der vorliegenden Erfindung dichtet der Schlauchabschnitt den Spalt zwischen Außen- und Innenkanüle hinreichend ab, wenn keine signifikanten Mengen von Atemluft und/oder Sekret durch den abgedichteten Spalt hindurchströmen bzw. fließen kann. Im Vordergrund steht das Ziel, bei einem abgedichteten Spalt neben der wirksamen Beatmung, die durch die Innenkanüle erfolgt, ein Eindringen von Sekret aus den oberen Atemwegen oder auch von Feuchtigkeit oder Krankheitskeimen durch einen proximalen Spalt zwischen Außen-und Innenkanüle zu verhindern, die ansonsten Entzündungen der unteren Atemwege hervorrufen könnten.

Sind die Enden des radial verformbaren Schlauchabschnittes mit der Innenkanüle dicht verbunden, kann der Schlauchabschnitt zur Abdichtung der in die Außenkanüle eingesetzten Innenkanüle radial nach außen aufgebläht und so an die Innenfläche der Außenkanüle angelegt werden. Dieser Form ist nicht beansprucht.

In einer Ausführungsform liegt der radial bewegbare Bereich des Schlauchabschnittes in seinem zweiten Zustand vollumfänglich an der Außenfläche der in die Außenkanüle eingeführten Innenkanüle an.

Ersetzt der Schlauchabschnitt einen Teil der Außen- oder Innenkanüle, kann der radial bewegbare Bereich des Schlauchabschnittes in seinem ersten Zustand denselben Innen oder Außendurchmesser wie die jeweilige Kanüle haben, deren Teil der Schlauchabschnitt ist, und hat ein radiales Spiel gegenüber der jeweils anderen Kanüle, während der Schlauchabschnitt in einem zweiten Zustand vollumfänglich an der Außen- oder Innenfläche der jeweils anderen Kanüle anliegt.

Mit dem Schlauchabschnitt in dem ersten Zustand kann die Innenkanüle problemlos durch die proximale Öffnung in die Außenkanüle ein- und ausgeführt werden ohne dass der Schlauchabschnitt die Reibung zwischen der Außenkanüle und der Innenkanüle wesentlich erhöht.

Nach dem Einführen der Innenkanüle in die Außenkanüle kann der Schlauchabschnitt aus dem ersten Zustand in den zweiten Zustand überführt werden, sodass der Spalt zwischen der Außen-und Innenkanüle durch den Schlauchabschnitt abgedichtet wird.

Der Schlauchabschnitt kann ein Teilabschnitt der Außenkanüle sein. Durch eine radiale Verformung des Schlauchabschnittes verkleinert sich der Innenquerschnitt der Außenkanüle in dem Bereich des Schlauchabschnittes, sodass der Spalt zwischen der Außen- und Innenkanüle verschlossen wird.

Zweckmäßigerweise kann der Schlauchabschnitt aus dem gleichen Material wie die Außenkanüle oder aus einem anderen Material hergestellt sein, ist aber leichter elastisch verformbar. Ist der Schlauchabschnitt und die Außenkanüle aus dem gleichen Material hergestellt, sind der Schlauchabschnitt und die Außenkanüle in einer Ausführungsform einstückig miteinander verbunden. Der Schlauchabschnitt und die Außenkanüle können jedoch auch mit Hilfe eines Fügeverfahrens miteinander verbunden sein. Vorzugsweise sind der Schlauchabschnitt und die Außenkanüle miteinander verklebt oder verschweißt.

Ist der Schlauchabschnitt ein Teilabschnitt der Außenkanüle, kann der Schlauchabschnitt gegenüber den hierzu distalen und proximalen Abschnitten der Außenkanüle insbesondere eine reduzierte Wandstärke aufweisen. Bedingt durch die reduzierte Wandstärke lässt sich der Schlauchabschnitt im Vergleich zu den distalen und proximalen Abschnitten der Außenkanüle leichter verformen, um so den Schlauchabschnitt aus dem ersten Zustand in den zweiten Zustand zu überführen, und umgekehrt.

In einer Ausführungsform ist der Schlauchabschnitt mit seinen proximalen und distalen Enden an der Innenseite der Außenkanüle dicht fixiert und die Wand der Außenkanüle weist in dem Bereich zwischen den proximalen und distalen Enden des Schlauchabschnittes eine Unterbrechung oder Perforation auf, sodass der Schlauchabschnitt von der Außenseite der Außenkanüle her mit einem Druck beaufschlagbar und dadurch radial nach innen bewegbar ist.

Das proximale und distale Ende des Schlauchabschnittes bilden jeweils einen Befestigungsrand der in Umfangsrichtung unter Aussparung eins zentralen Schlauchabschnittes dicht mit der Innenseite der Außenkanüle verbunden ist. Der zwischen dem proximalen Befestigungsrand und dem distalen Befestigungsrand angeordnete, zentrale Schlauchabschnitt bildet zusammen mit der ihm zugewandten Innenfläche der Außenkanüle einen Hohlraum der über die Unterbrechung oder Perforation mit einem Druck beaufschlagbar ist. Wird der Hohlraum beispielweise mit einem Fluid befüllt, hebt sich der zentrale Abschnitt von der Innenfläche der Außenkanüle ab, verformt und bewegt sich in radialer Richtung auf die Innenkanüle zu. Bei steigendem Druck schmiegt sich der zentrale Abschnitt in Umfangsrichtung an die Außenfläche der Innenkanüle und dichtet so den Spalt zwischen Außen- und Innenkanüle ab.

Wird das Fluid aus dem Hohlraum abgelassen, sinkt der Druck und der zentrale Abschnitt bewegt sich in radialer Richtung zurück in seinen Ausgangszustand, das ist der erste Zustand. Die Innenkanüle kann aus der Außenkanüle entfernt werden.

Die Unterbrechung kann ein ringförmiger Spalt mit einer lichten, im ersten Zustand gemessenen Spaltbreite d von mindestens 0,2 mm und maximal 1 mm sein. Bevorzugt sind jedoch eine oder mehrere radiale Öffnungen bzw. Perforationen in der Wand der Außenkanüle, die sich allerdings auf den axialen Bereich des Schlauchabschnitts beschränken.

Zweckmäßigerweise kann eine Überführung des Schlauchabschnittes aus dem ersten Zustand in den zweiten Zustand, und umgekehrt, von außen, d.h. von dem proximalen Ende der Trachealbeatmungsvorrichtung aus, bewirkt werden.

Erfindungsgemäss weist die Außenkanüle einen Cuff zur Abdichtung und Fixierung der Außenkanüle in der Trachea auf. Ein radial bewegbarer Schlauchabschnitt liegt dann axial zwischen den mit der Außenkanüle verbundenen distalen und proximalen Enden des Cuffs und wird so mit dem Druck des Cuffs beaufschlagt.

Der auf der Außenseite des Kanülenrohrs angeordnete Cuff dient der Abdichtung und weichen Fixierung des Kanülenrohrs in einer Trachea und besteht üblicherweise aus einem aufblasbaren, sehr dünnwandigen Schlauch. In Umfangsrichtung der Außenkanüle ist der Cuff mit einem proximalen Befestigungsrand und einem distalen Befestigungsrand fluiddicht gegenüber der Außenfläche der Außenkanüle abgedichtet. Der proximale Befestigungsrand und der distale Befestigungsrand sind die Endabschnitte des Schlauches und fest mit der Außenkanüle verbunden. Mindestens in einem zentralen Abschnitt zwischen den Befestigungsrändern hat der Cuff ein deutliches Übermaß gegenüber der Außenkanüle oder ist auf ein solches Übermaß aufblasbar bzw. mit einem Fluid befüllbar. Nach dem Einführen der Außenkanüle und Befüllen des Cuffs mit einem Fluid legt sich der Cuff, vorzugsweise mit einem geringen Druck, an die Wand der Trachea entlang seines Umfangs abdichtend an.

Der Schlauchabschnitt ist in einer Variante axial und radial innerhalb des vom Cuff in Anspruch genommenen Bereichs an der Innenseite der an dieser Stelle perforierten Außenkanüle angeordnet oder wird durch eine dünnen und elastisch leicht verformbaren Wandabschnitt der Außenkanüle gebildet. Durch eine Änderung des Füllstandes des Cuff kann der Schlauchabschnitt aus seinem ersten Zustand in den zweiten Zustand überführt werden, und umgekehrt. Hierzu wird der Cuff mit einem Fluid befüllt und so der Druck in dem Cuff erhöht. Der Cuff dehnt sich aus und der Cuffdruck wirkt auch auf den Schlauchabschnitt. Der elastische Schlauchabschnitt verformt sich und wölbt sich in Richtung der Innenkanüle bis er sich zur Abdichtung der Innenkanüle in der Außenkanüle an der Außenfläche der Innenkanüle anschmiegt. In diesem zweiten Zustand ist der Spalt zwischen der Außenkanüle und der Innenkanüle durch den Schlauchabschnitt dicht verschlossen. Atemgas und/oder Sekret können an dem Schlauchabschnitt in seinem zweiten Zustand nicht vorbeigelangen.

Zum Überführen des Schlauchabschnittes aus dem zweiten Zustand in den ersten Zustand wird der Füllstand in dem Cuff reduziert. Das Volumen des Cuffs verkleinert sich und der elastische Schlauchabschnitt kehrt in seinen Ausgangszustand, d.h. in den ersten Zustand, zurück.

Zu Erhöhung und Absenkung des Füllstandes ist der Cuff mit einem Füllschlauch und einer Füllvorrichtung verbindbar. Mit einer Erhöhung des Füllstandes kann der Druck in dem Cuff erhöht werden. Entsprechend kann durch eine Absenkung des Füllstandes der Druck in dem Cuff gesenkt werden.

In einer Ausführungsform steht die Unterbrechung oder Perforation mit dem Inneren des Cuffs in Verbindung. Wird der Cuff mit Fluid befüllt, gelangt das Fluid durch die Unterbrechung oder Perforation in den von dem zentralen Schlauchabschnitt und der Innenfläche der Außenkanüle gebildeten Hohlraum. Der Hohlraum dehnt sich in Folge der Fluidaufnahme aus, indem sich der zentrale Schlauchabschnitt radial verformt und sich in radialer Richtung auf die Innenkanüle zubewegt. Wird das Fluid aus dem Cuff abgelassen, schrumpft der Hohlraum und der Schlauchabschnitt kehrt in den ersten Zustand zurück.

In einer Ausführungsform beträgt der Cuffinnendruck in dem zweiten Zustand 20 hPa bis 30 hPa. Der Schlauchabschnitt kann Teil der Hülle des Cuffs sein. Wird der Cuff mit einem Fluid befüllt dehnt sich der Schlauchabschnitt aus und dichtet so den Spalt zwischen der Außenkanüle und der Innenkanüle ab.

Ein zwischen einem Schlauchabschnitt und einer Wand der Innen- oder Außenkanüle gebildeter Hohlraum kann jedoch auch durch eine separate Zuleitung (zum Beispiel einem in die jeweilige Wand integrierten dünnen Kanal) vom Cuff unabhängig separat befüllbar sein. Da der Schlauchabschnitt nur zwischen Außen und Innenkanüle bewegbar ist und dort abdichtet, ist der Druck in diesem Schlauchabschnitt viel weniger kritisch als der Druck in einem Cuff und kann mit weniger Aufwand bereitgestellt werden und auch die Wandstärke des Schlauchabschnitts kann wesentlich größer sein als die eines Cuffs und zum Beispiel zwischen 10 und 450 µm. betragen. Dieser Ausführungsform wird nicht beansprucht.

Damit der Schlauchabschnitt nicht ungewollt das Ein- und Ausführen der Innenkanüle in bzw. aus der Außenkanüle erschwert, ist in einer Ausführungsform vorgesehen, dass der Schlauchabschnitt an einem radial nach außen rückspringenden Abschnitt der Innenwand der Außenkanüle angeordnet ist. In dem nach außen rückspringenden Abschnitt kann der Schlauchabschnitt in dem ersten Zustand zumindest teilweise aufgenommen werden, sodass der Schlauchabschnitt beim Ein- und Ausführen der Innenkanüle nicht ungewollt zwischen der Außen- und Innenkanüle eingeklemmt wird.

In einer Ausführungsform beträgt das durch den Schlauchabschnitt zu überbrückende maximale radiale Spiel zwischen Innenkanüle und Schlauchabschnitt in dem ersten Zustand zwischen 0.2 mm und 1 mm.

In dem ersten Zustand und in dem zweiten Zustand definiert der Schlauchabschnitt jeweils einen minimalen Innendurchmesser (ID1, ID2), wobei in einer Ausführungsform der minimale Innendurchmesser (ID2) in dem zweiten Zustand, vorzugsweise 0,4 mm bis 2 mm, besonders bevorzugt 0,6 mm bis 1 mm, kleiner als der minimale Innendurchmesser (ID2) in dem ersten Zustand ist. Zweckmäßigerweise kann der minimale Innendurchmesser ID2 in dem zweiten Zustand dem Außendurchmesser des radialinnenliegend zu dem Schlauchabschnitt angeordneten Bereichs der Innenkanüle entsprechen. In dem ersten Zustand kann der minimale Innendurchmesser des Schlauchabschnittes größer oder gleich dem mittleren Innendurchmesser der Außenkanüle in dem von dem proximalen Befestigungsrand des Cuffs und dem distalen Befestigungsrand begrenzten Bereich der Außenkanüle sein.

Der elastisch verformbare Schlauchabschnitt kann in seinem ersten Zustand zwischen seinen befestigten proximalen und distalen Enden eine axiale Länge von 5 mm bis 50 mm, vorzugsweise von 5 mm bis 20 mm, aufweisen. Die axiale Länge des Schlauchabschnittes in seinem ersten Zustand ist ein Parameter, der mitbeeinflusst wie weit sich der Schlauchabschnitt in dem zweiten Zustand in das Lumen der Außenkanülen hineinerstrecken bzw. über welche axiale Länge sich der Schlauchabschnitt an die Außenfläche der Innenkanüle anlegen kann.

Die elastische Verformbarkeit des Schlauchabschnittes kann durch die Auswahl des Kunststoffes und der mittleren Wandstärke mitbeeinflusst werden, wobei in einer Ausführungsform der Schlauchabschnitt aus einem Kunststoff mit einer Shore-Härte im Bereich von A50 bis D50 und einer mittleren Wandstärke von 0,05 bis 0,5 mm hergestellt ist.

In einer Ausführungsform ist der Cuff ein High-Volume-Low-Pressure-Cuff ist.

Die Außenkanüle kann ebenso wie die Innenkanüle eine Phonationsöffnung, insbesondere eine Fensterung oder eine Siebung, aufweisen, die sich bezüglich des Cuffs und des Schlauchabschnittes an den proximalen Abschnitten von Außen- und Innenkanüle befinden.

Weitere Vorteile, Merkmale und Anwendungsmöglichkeiten der vorliegenden Erfindung werden anhand der vorliegenden Beschreibung von Ausführungsformen und der dazugehörigen Figuren deutlich, wobei gleiche Bezugszeichen auf gleiche Elemente verweisen. Es zeigen:
- Figur 1a: eine Seitenansicht eines Trachealbeatmungsvorrichtung nach einer ersten Ausführungsform der Erfindung, wobei der Schlauchabschnitt in dem ersten Zustand ist,
- Figur 1b: eine Schnittansicht entlang der Schnittlinie A-A durch die Ausführungsform aus Figur 1a,
- Figur 2a: eine Seitenansicht des Trachealbeatmungsvorrichtung gemäß der ersten Ausführungsform der Erfindung, wobei der Schlauchabschnitt in dem zweiten Zustand ist,
- Figur 2b: eine Schnittansicht entlang der Schnittlinie D-D durch die Ausführungsform aus Figur 2a,
- Figur 3a: einen vergrößerten Teilschnitt aus der Figur 1b zur Veranschaulichung des Schlauchabschnittes in dem ersten Zustand,
- Figur 3b: einen vergrößerten Teilschnitt aus der Figur 2b zur Veranschaulichung des Schlauchabschnittes in dem zweiten Zustand,
- Figur 4a: eine Seitenansicht einer Trachealbeatmungsvorrichtung gemäß einer zweiten Ausführungsform der vorliegenden Erfindung zur Veranschaulichung ohne in die Außenkanüle eingesetzte Innenkanüle und mit dem Schlauchabschnitt in dem ersten Zustand,
- Figur 4b: eine Schnittansicht entlang der Linie A-A durch die Ausführungsform der Figur 4a,
- Figur 5a: eine Seitenansicht des Trachealbeatmungsvorrichtung gemäß der zweiten Ausführungsform zur Veranschaulichung ohne in die Außenkanüle eingesetzte Innenkanüle und mit dem Schlauchabschnitt in dem zweiten Zustand,
- Figur 5b: eine Schnittansicht entlang der Linie A-A durch die Ausführungsform gemäß der Figur 5a,
- Figur 6a: eine Seitenansicht eines Trachealbeatmungsvorrichtung gemäß einer dritten Ausführungsform der vorliegenden Erfindung zur Veranschaulichung ohne in die Außenkanüle eingesetzte Innenkanüle und mit dem Schlauchabschnitt in dem zweiten Zustand,
- Figur 6b: eine Schnittansicht entlang der Linie A-A durch die Ausführungsform gemäß der Figur 6a,
- Figur 6c: einen vergrößerten Teilschnitt aus der Figur 6b zur Veranschaulichung des Schlauchabschnittes in seinem zweiten Zustand.

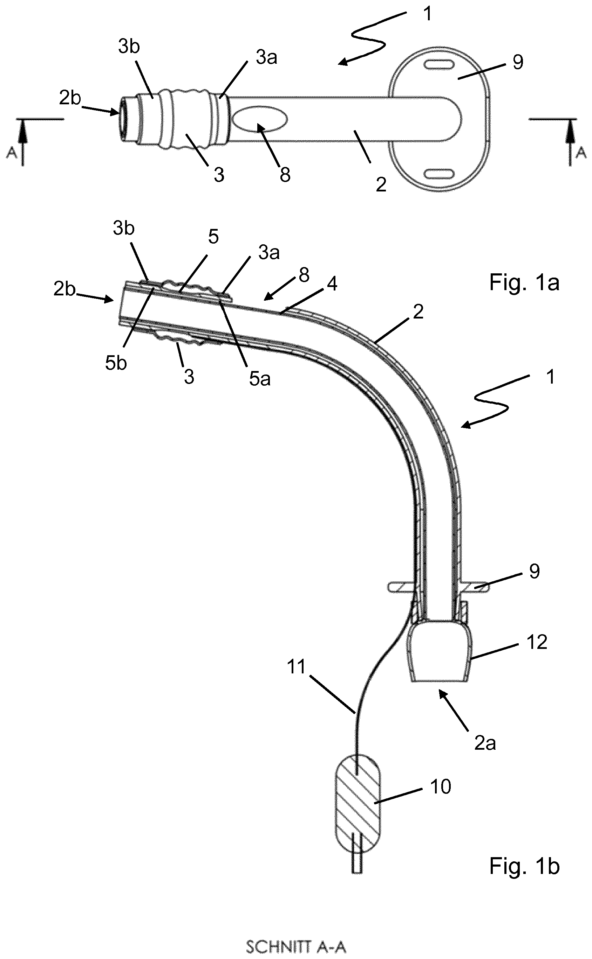

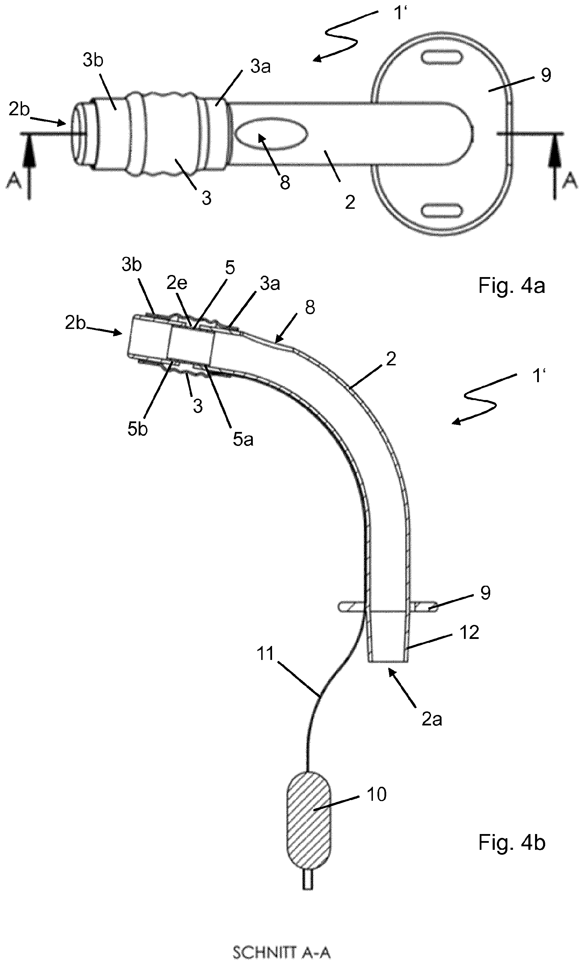

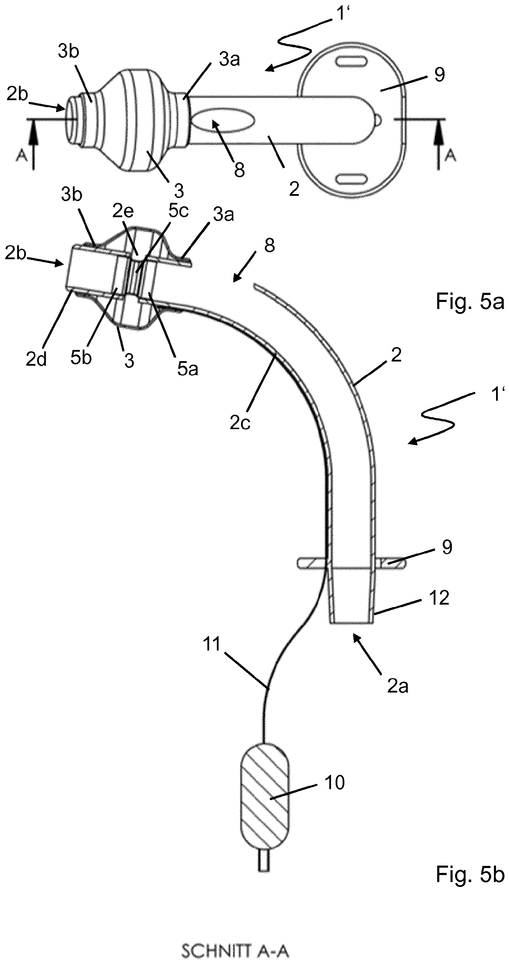

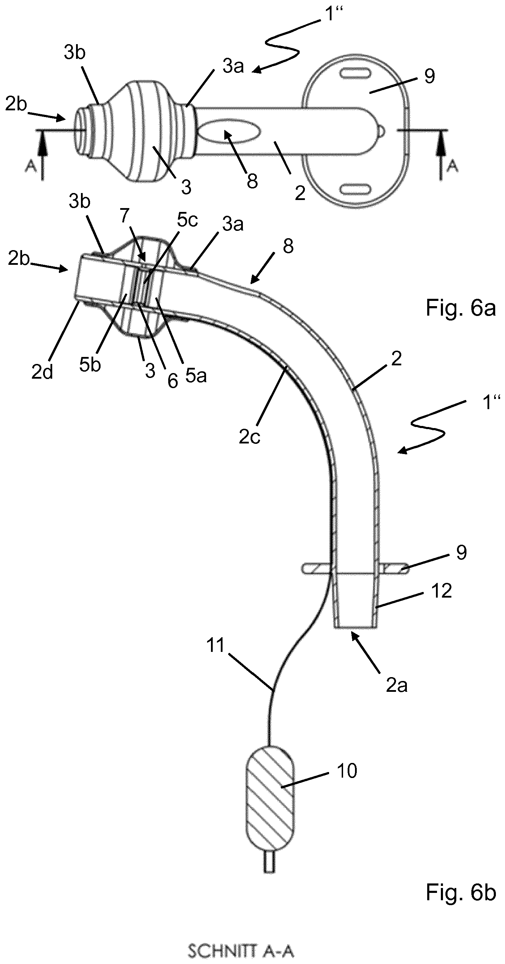

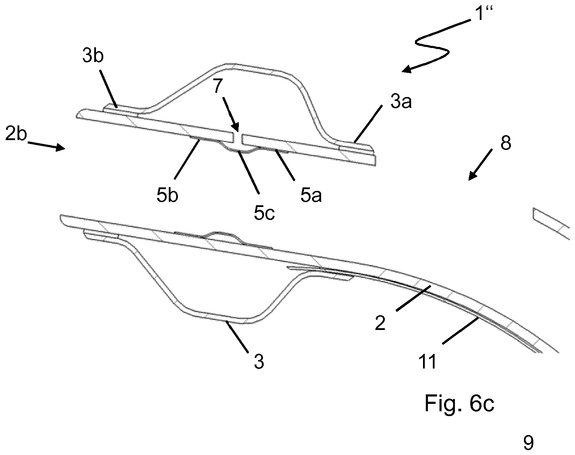

In den Figuren sind verschiedene Ausführungsformen einer Trachealbeatmungsvorrichtung 1, 1', 1" gemäß der vorliegenden Erfindung dargestellt. Die Figuren 1a, 1b, 2a, 2b, 3a und 3b zeigen eine erste Ausführungsform einer Trachealbeatmungsvorrichtung 1, die Figuren 4a, 4b, 5a und 5b zeigen eine zweite Ausführungsform einer Trachealbeatmungsvorrichtung 1' und die Figuren 6a, 6b und 6c zeigen eine dritte Ausführungsform einer Trachealbeatmungsvorrichtung 1".

Alle Ausführungsformen zeigen eine Trachealbeatmungsvorrichtung 1,1',1" mit einer Außenkanüle 2. Die Außenkanüle 2 hat eine proximale Öffnung 2a und eine distale Öffnung 2b und dient der Hindurchleitung von Atemgas. Am proximalen Ende der Außenkanüle 2 ist ein Kanülenschild 9 angeordnet, das zur Befestigung der Trachealbeatmungsvorrichtung mit einem nicht dargestellten, um den Hals eines Patienten herumführbaren Kanülenbands verbindbar ist. Das Kanülenschild 9 ist kein notwendiges Merkmal der vorliegenden Erfindung.

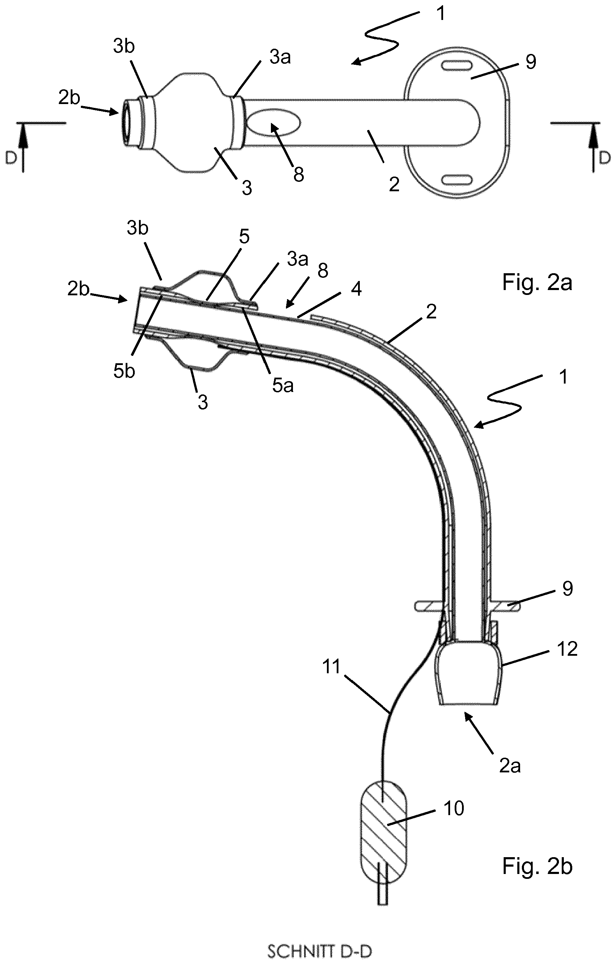

Auf seiner Außenseite weist die Außenkanüle 2 einen Cuff 3 auf. Der Cuff 3 besteht aus einem aufblasbaren, sehr dünnwandigen Schlauch, dessen zwei Endabschnitte außen auf der Außenkanüle 2 eng anliegen und dort befestigt sind. Die Endabschnitte bilden einen proximalen Befestigungsrand 3a und einen distalen Befestigungsrand 3b, die den Cuff 3 in Umfangsrichtung gegenüber der Außenkanüle 2 fluiddicht abschließen. Der Cuff 3 dient in seinem befüllten Zustand der Abdichtung der Außenkanüle 2 in einer Trachea. Zu seiner Befüllung und Entleerung ist der Cuff 3 in der gezeigten Ausführungsform über einen Füllschlauch 11 mit einer Füllvorrichtung 10, wie beispielsweise ein Kontrollballon mit Ventil, verbunden. Am proximalen Ende der Innen- oder Außenkanüle befindet sich ein sogenannter 15 mm Konnektor 12 zum Anschluss an den Beatmungsschluss. Beispielhaft ist in den Figuren 1b und 2b die Innenkanüle mit dem 15 mm Konnektor 12 dargestellt, während in den Figuren 4b, 5b und 6b die Außenkanüle den 15 mm Konnektor 12 aufweist. Der Füllschlauch 11, die Füllvorrichtung 10 und der Konnektor 12 sind als solche kein notwendiges Merkmal der vorliegenden Erfindung.

Um einem Patienten trotz in die Trachea eingeführter Trachealbeatmungsvorrichtung 1, 1', 1" das Sprechen zu ermöglichen, weist die Außenkanüle 2 der gezeigten Ausführungsformen proximal von dem Cuff 3 eine Phonationsöffnung 8 auf. Durch die Phonationsöffnung 8 kann Atemgas aus der Außenkanüle 2 über die Stimmbänder in den natürlichen Atemwegen geleitet werden. Auch die Phonationsöffnung 8 ist kein notwendiges Merkmal der Erfindung.

Zum zeitweisen Verschließen der Phonationsöffnung 8 oder aber aus anderen Gründen ist eine Innenkanüle 4 vorgesehen. Der Innenquerschnitt der Außenkanüle 2 und der Außenquerschnitt der Innenkanüle 4 sind derart aufeinander abgestimmt, dass die Innenkanüle 4 durch die proximale Öffnung 2a in die Außenkanüle 2 einführbar ist.

Die Innenkanüle 4 dient der Durchleitung von Atemluft und sollte zur Minimierung des Atemwiderstands ein möglichst großes Lumen aufweisen. Andererseits sollte sie sich auch leicht in die Außenkanüle einsetzen bzw. daraus entnehmen lassen. Dazu ist ein gewisses Spiel zwischen der Außenkanüle 2 und der Innenkanüle 4 erforderlich.

Damit die in die Außenkanüle 2 eingesetzte Innenkanüle 4 gegenüber der radialaußenliegenden Außenkanüle 2 abgedichtet werden kann, weist die Außenkanüle 2 distal von der Phonationsöffnung 8 einen radial verformbaren Schlauchabschnitt 5 auf.

Der Schlauchabschnitt 5 ist radial innenliegend zu dem Cuff 3 in einem Bereich der Außenkanüle 2 angeordnet, der von dem proximalen Befestigungsrand 3a und dem distalen Befestigungsrand 3b begrenzt ist. Durch eine Änderung des Füllstandes des Cuffs ist der radial verformbare Schlauchabschnitt 5 von einem ersten Zustand, wie er nachfolgend im Zusammenhang mit den Figuren 1a, 1b, 3a, 4a und 4b beschrieben ist, in einen zweiten Zustand, wie er nachfolgend im Zusammenhang mit den Figuren 2a, 2b, 3b, 5a, 5b, 6a, 6b und 6c beschrieben ist, überführbar, und umgekehrt.

In dem zweiten Zustand ist der Fülldruck des Cuffs größer als der Fülldruck des Cuffs in dem ersten Zustand. Der Schlauchabschnitt 5 kann dementsprechend aus dem ersten Zustand durch eine Erhöhung des Fülldrucks des Cuffs in den zweiten Zustand überführt werden. Sinkt der Fülldruck in dem Cuff wieder, wird der Schlauchabschnitt 5 aus dem zweiten Zustand in den ersten Zustand überführt.

In dem zweiten Zustand führt der erhöhte Druck in dem Cuff 3 dazu, dass der Schlauchabschnitt 5 gegen die Außenfläche der in die Außenkanüle 2 eingeführten Innenkanüle 4 drückt und abdichtet. Der an der Außenfläche der Innenkanüle 4 zur Abdichtung anliegende Schlauchabschnitt 5 verhindert, dass Atemluft und/oder Sekret zwischen der Außenkanüle 2 und der Innenkanüle 4 an dem Schlauchabschnitt 5 in einer nennenswerten Menge vorbeigelangt. In dem ersten Zustand liegt der Schlauchabschnitt 5 hingegen zumindest nicht vollständig an der Außenfläche der in die Außenkanüle 2 eingeführten Innenkanüle 4 an. Atemgas und/oder Sekret können zwischen der Außenkanüle 2 und der Innenkanüle 4 an dem Schlauchabschnitt 5 vorbeigelangen. Der erste Zustand dient dem Einbringen der Innenkanüle 4 in die Außenkanüle 2 bzw. dem Entfernen der Innenkanüle 4 aus der Außenkanüle 2.

Die in den Figuren dargestellten Ausführungsformen unterscheiden sich lediglich in der Ausgestaltung des Schlauchabschnittes 5 sowie in der Ausgestaltung der Außenkanüle 2 in dem durch den proximalen Befestigungsrand 3a des Cuffs 3 und den distalen Befestigungsrand 3b des Cuffs 3 begrenzten Bereich.

Gemäß der ersten Ausführungsform ist der Schlauchabschnitt 5 aus einem Kunststoff mit einer Shore-Härte von D40 und einer mittleren Wandstärke von 0,3 mm hergestellt. Dabei erstreckt sich der Schlauchabschnitt 5 in seinem ersten Zustand, wie er in den Figuren 1a und 1b dargestellt ist, über eine Länge in axialer Richtung der Außenkanüle 2 von 5 mm bis 20 mm. Der Schlauchabschnitt 5 ist ein Abschnitt der Außenkanüle 2 mit einer reduzierten Wandstärke und ersetzt bei dieser Ausführungsform einen Teil der Außenkanüle 2. Wird der radialaußenliegend zu dem Schlauchabschnitt 5 angeordnete Cuff 3 mit einem Fluid befüllt, dehnt sich der Cuff 3 aus und der Luftdruck drückt gegen den Schlauchabschnitt 5. Erreicht der Druck in dem Cuff einen Bereich von 20 hPa bis 30 hPa ist der Schlauchabschnitt 5 in dem zweiten Zustand, in dem der Schlauchabschnitt 5 radial verformt und vollumfänglich gegen die Außenfläche der Innenkanüle 4 zum Abdichten des Spalts zwischen Außen- und Innenkanüle gegen die Außenfläche der Innenkanüle 4 gepresst ist. Der Anpressdruck des Schlauchabschnittes 5 gegen die Innenkanüle 4 ist so gering, dass es zu keiner unerwünschten Verformung der Innenkanüle 4 kommt. Das Ziel einer ausreichenden Abdichtung wird hingegen erreicht.

Befindet sich die Außenkanüle 2 in einer Trachea, in den Figuren 2a und 2b nicht gezeigt, drückt der befüllte Cuff 3 in dem zweiten Zustand des Schlauchabschnittes 5 gleichzeitig gegen die Trachea und dichtet die Außenkanüle 2 gegenüber der Trachea ab.

Wird der Druck in dem Cuff 3 auf unter 20 hPa abgesenkt, kehrt der Schlauchabschnitt 5 sukzessive in Richtung des ersten Zustands zurück. In dem ersten Zustand, wie er in den Figuren 2a und 2b dargestellt ist, liegt der Schlauchabschnitt 5 zumindest nicht vollumfänglich an der Außenfläche der in die Außenkanüle 2 eingesetzten Innenkanüle 4 an. Atemgas und/oder Sekret können zwischen der Außenkanüle 2 und der Innenkanüle 4 an dem Schlauchabschnitt 5 vorbeigelangen.

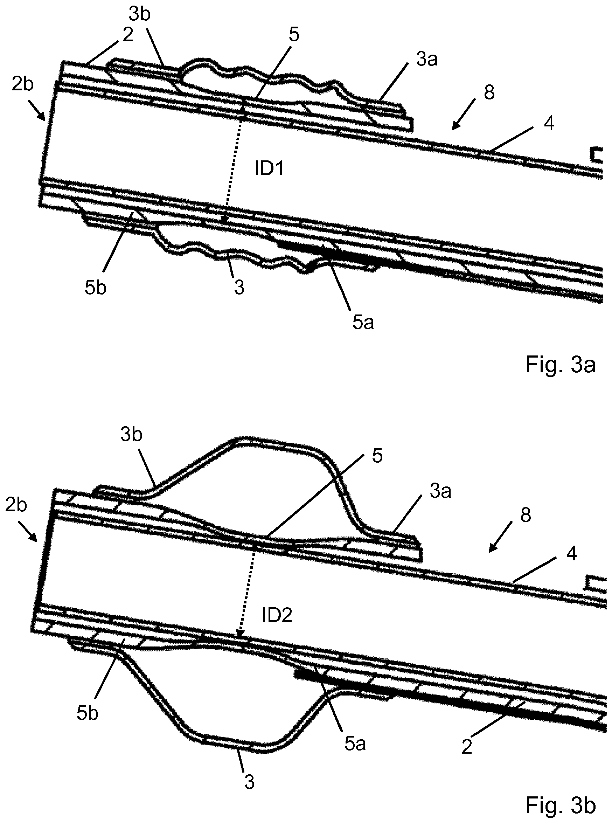

Eine vergrößerte Schnittansicht, die den Schlauchabschnitt 5 der ersten Ausführungsform in seinem ersten Zustand zeigt, ist in der Figur 3a dargestellt. Gut erkennbar weist der Schlauchabschnitt 5 eine im Vergleich zu den hierzu distalen und proximalen Abschnitten der Außenkanüle 2 eine reduzierte Wandstärke auf.

Eine vergrößerte Schnittansicht, die den Schlauchabschnitt 5 der ersten Ausführungsform in seinem zweiten Zustand zeigt, ist in der Figur 3b dargestellt. Gut erkennbar ist der Schlauchabschnitt 5 durch den Druck des befüllten Cuffs 3 verformt und wird gegen die Außenfläche der in der Außenkanüle 2 angeordneten Innenkanüle 4 zum Abdichten der Innenkanüle 4 in der Außenkanüle 2 gedrückt.

Der minimale Innendurchmesser ID2 des Schlauchabschnitts 5 in dem zweiten Zustand ist kleiner als der minimale Innendurchmesser ID1 des Schlauchabschnittes 5 in dem ersten Zustand. Der minimale Innendurchmesser ID2 des Schlauchabschnittes 5 in dem zweiten Zustand entspricht dem Außendurchmesser des radialinnenliegend zu dem Schlauchabschnitt 5 angeordneten Bereichs der Innenkanüle 4 und ist um 0,4 mm bis 2 mm kleiner als der der minimale Innendurchmesser ID1 des Schlauchabschnittes 5 in dem ersten Zustand.

Die Außenkanüle 2 der zweiten, in den Figuren 4a, 4b, 5a und 5b gezeigten Ausführungsform weist eine Unterbrechung in Form eines ringförmigen Spaltes 2e mit der lichten Spaltbreite d auf. Der Spalt 2e unterteilt die Außenkanüle 2 in einen proximalen Rohrabschnitt 2c und einen distalen Rohrabschnitt 2d, sodass der proximale Rohrabschnitt 2c und der distale Rohrabschnitt 2d in dem ersten Zustand des Schlauchabschnitts 5 entsprechend der Spaltbreite d voneinander beabstandet sind. Zur besseren Darstellung des Schlauchabschnitts 5 ist die Innenkanüle 4 in den Figuren 4a, 4b, 5a und 5b nicht dargestellt.

Der Schlauchabschnitt 5 ist Teil der Hülle des Cuffs 3 und überdeckt den ringförmigen Spalt 2e, wobei der Schlauchabschnitt 5 gemäß der zweiten Ausführungsform einen proximalen Befestigungsrand 5a, einen distalen Befestigungsrand 5b und einen zentralen Abschnitt 5c aufweist. Der proximale Befestigungsrand 5a ist fluiddicht unter Aussparung des zentralen Abschnitts 5c mit dem proximalen Rohrabschnitt 2c und der distale Befestigungsrand 5b ist fluiddicht unter Aussparung des zentralen Abschnitts 5c mit dem distalen Rohrabschnitt 2d verbunden. Genauer ist der proximale Befestigungsrand 5a in Umfangsrichtung mit der Innenfläche des proximalen Rohrabschnittes 2c und der distale Befestigungsrand 5b in Umfangsrichtung mit der Innenfläche des distalen Rohrabschnittes 2d verklebt.

Die Figur 4b zeigt den Schlauchabschnitt 5 in seinem ersten Zustand. Der Cuff 3 ist weitestgehend leer, sodass der elastische Schlauchabschnitt 5 nicht verformt ist. Wird der Cuff 3 mit einem Fluid befüllt, steigt der Druck in dem Cuff 3 an. Der Schlauchabschnitt 5 beginnt sich radial zu verformen und wölbt sich in Richtung der Innenkanüle, die in den Figuren 4b und 5b nicht dargestellt ist. Bei einem Druck von 20 hPa bis 30 hPa befindet sich der Schlauchabschnitt 5 in seinem zweiten Zustand, wie er in der Figur 5b gezeigt ist. Der minimale Innendurchmesser ID2 des Schlauchabschnittes 5 in dem zweiten Zustand ist kleiner als der minimale Innendurchmesser ID1 des Schlauchabschnittes 5 in dem ersten Zustand.

Die Figuren 6a, 6b und 6c zeigen den Schlauchabschnitt 5 der Trachealbeatmungsvorrichtung 1" in dem zweiten Zustand gemäß einer dritten Ausführungsform, wobei zugunsten einer besseren Darstellung des Schlauchabschnittes 5 auf eine Abbildung der in die Außenkanüle 2 eingesetzten Innenkanüle 4 verzichtet wurde.

Der Schlauchabschnitt 5 weist einen proximalen Befestigungsrand 5a, einen distalen Befestigungsrand 5b und einen zentralen Abschnitt 5c auf. Der proximale Befestigungsrand 5a und der distale Befestigungsrand 5b sind jeweils unter Aussparung des zentralen Abschnittes 5c in Umfangsrichtung fluiddicht mit der Innenfläche der Außenkanüle 2 verklebt. Der zentrale Abschnitt 5c definiert mit den von ihm abgedeckten Teil der Innenfläche der Außenkanüle 2 einen Hohlraum 6, der über eine Perforation 7 in fluider Verbindung mit dem Cuff 3 steht.

Wird der Cuff 3 mittels der Füllvorrichtung 10 und den Füllschlauch 11 mit einem Fluid befüllt, gelangt dieses Fluid durch die mindestens eine Perforation 7 auch in den Hohlraum 6 zwischen dem zentralen Abschnitt 5c und der Innenfläche der Außenkanüle 2. Durch die Beaufschlagung mit Fluid nimmt das Volumen des Hohlraums 6 zu, sodass der Schlauchabschnitt 5 von seinem ersten Zustand in den zweiten Zustand übergeht. In dem zweiten Zustand ist der minimale Innendurchmesser ID2 des Schlauchabschnittes 5 kleiner als der minimale Innendurchmesser ID1 in dem ersten Zustand.

Das Volumen zwischen der Innenwand der Außenkanüle 2 und dem bewegbaren Bereich 5c des Schlauchabschnittes 5 könnte jedoch auch durch einen separaten Füllschlauch ähnlich dem Füllschlauch 11 für den Cuff 3, befüllt werden. Der Fülldruck und die erforderliche Wandstärke des Schlauchabschnittes 5 wären dann unabhängig von dem Cuff 3. Bei einer solchen Variante könnte der Schlauchabschnitt 5 auch ebenso gut an der Außenseite einer Innenkanüle 4 angebracht sei. Insbesondere kann ein solcher Füllschlauch auch in die Wand von Außen- bzw. Innenkanüle integriert werden, zumal das Volumen des Hohlraumes zwischen Schlauchabschnitt 5 und Wand von Außen- bzw. Innenkanüle sehr klein gehalten werden kann, wie man auch anhand der Figur 6c erkennt.

Für Zwecke der ursprünglichen Offenbarung wird darauf hingewiesen, dass sämtliche Merkmale, wie sie sich aus der vorliegenden Beschreibung, den Zeichnungen und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarerer Merkmalskombinationen wird hier nur der Kürze und der Lesbarkeit der Beschreibung wegen verzichtet.

Während die Erfindung im Detail in den Zeichnungen und der vorangehenden Beschreibung dargestellt und beschreiben wurde, erfolgt diese Darstellung und Beschreibung lediglich beispielhaft und ist nicht als Beschränkung des Schutzbereichs gedacht, so wie er durch die Ansprüche definiert wird. Die Erfindung ist nicht auf die offenbarten Ausführungsformen beschränkt.

Abwandlungen der offenbarten Ausführungsformen sind für den Fachmann aus den Zeichnungen, der Beschreibung und den beigefügten Ansprüchen offensichtlich. In den Ansprüchen schließt das Wort "aufweisen" nicht andere Elemente oder Schritte aus, und der unbestimmte Artikel "eine" oder "ein" schließt eine Mehrzahl nicht aus. Die bloße Tatsache, dass bestimmte Merkmale in unterschiedlichen Ansprüchen beansprucht sind, schließt ihre Kombination nicht aus. Bezugszeichen in den Ansprüchen sind nicht als Beschränkung des Schutzbereichs gedacht.

### Bezugszeichenliste

- 1: Trachealbeatmungsvorrichtung
- 2: Außenkanüle
- 2a: proximale Öffnung der Außenkanüle 2
- 2b: distale Öffnung der Außenkanüle 2
- 2c: proximaler Rohrabschnitt der Außenkanüle 2
- 2d: distaler Rohrabschnitt der Außenkanüle 2
- 2e: ringförmiger Spalt
- 3: Cuff
- 3a: proximaler Befestigungsrand des Cuffs 3
- 3b: distaler Befestigungsrand des Cuffs 3
- 4: Innenkanüle
- 5: Schlauchabschnitt zur Abdichtung der Innenkanüle 4 in der Außenkanüle 2
- 5a: proximaler Befestigungsrand des Schlauchabschnittes 5
- 5b: distaler Befestigungsrand des Schlauchabschnittes 5
- 5c: zentraler Abschnitt des Schlauchabschnittes 5
- 6: Hohlraum
- 7: Perforation
- 8: Phonationsöffnung
- 9: Kanülenschild
- 10: Füllvorrichtung für den Cuff 3
- 11: Füllschlauch
- 12: 15 mm Konnektor
- ID1: minimaler Innendurchmesser des Schlauchabschnitts 5 im ersten Zustand
- ID2: minimaler Innendurchmesser des Schlauchabschnitts 5 im zweiten Zustand
- d: Spaltbreite des ringförmigen Spaltes 2e

## Patentansprüche

1. Trachealbeatmungsvorrichtung (1), insbesondere Tracheostomiekanüle, mit einer Au-ßenkanüle (2) und einer Innenkanüle (4), wobei der Innenquerschnitt der Außenkanüle (2) und der Außenquerschnitt der Innenkanüle (4) derart aufeinander abgestimmt sind, dass die Innenkanüle (4) mit Spiel in der Außenkanüle (2) bewegbar ist, wobei die Außenkanüle (2) einen Cuff (3) zur Abdichtung und Fixierung der Außenkanüle (2) in der Trachea aufweist, wobei ein radial verformbarer Schlauchabschnitt (5) vorgesehen ist, dessen Enden (5a, 5b) mit der Außenkanüle (2) dicht verbunden sind, wobei ein Bereich des Schlauchabschnitts (5) zwischen dessen Enden (5a, 5b) aus einem ersten Zustand in einen zweiten Zustand radial bewegbar ist, und umgekehrt, wobei in dem zweiten Zustand der Schlauchabschnitt (5) die in die Außenkanüle (2) eingesetzte Innenkanüle (4) abdichtet, **dadurch gekennzeichnet, dass** der radial bewegbare Schlauchabschnitt (5) axial zwischen den mit der Außenkanüle (2) verbundenen distalen und proximalen Enden (3a, 3b) des Cuffs (3) liegt, wobei der Schlauchabschnitt (5) mit dem Druck des Cuffs beaufschlagt werden kann.

2. Trachealbeatmungsvorrichtung (1) nach Anspruch 1, **dadurch gekennzeichnet, dass** der der Schlauchabschnitt (5) dadurch mit dem Druck des Cuffs beaufschlagt werden kann, dass der Schlauchabschnitt (5) entweder
a) ein Teilabschnitt der Außenkanüle (2) ist, der gegenüber den hierzu distalen und proximalen Abschnitten der Außenkanüle (2) eine reduzierte Wandstärke aufweist, oder
b) Teil der Hülle des Cuffs (3) ist oder
c) mit seinen proximalen und distalen Enden (5a, 5b) an der Innenseite der Außenkanüle (2) dicht fixiert ist und die Wand der Außenkanüle (2) in dem Bereich zwischen diesen proximalen und distalen Enden (5a, 5b) eine Unterbrechung oder eine Perforation (7) aufweist, so dass der Schlauchabschnitt (5) von der Außenseite der Außenkanüle (2) her mit Druck beaufschlagbar ist, wobei die Perforation oder Unterbrechung (6) mit dem Inneren des Cuffs (3) in Verbindung steht.

3. Trachealbeatmungsvorrichtung (1) nach Anspruch 1 oder 2, **dadurch gekennzeichnet, dass** der radial bewegbare Bereich des Schlauchabschnittes (5) sich in einem ersten Zustand an die Innenfläche der Außenkanüle (2) anschmiegt und ein radiales Spiel gegenüber der Innenkanüle (4) hat und der radial bewegbare Bereich des Schlauchabschnittes (5) in einem zweiten Zustand vollumfänglich an der Außenfläche der in die Außenkanüle (2) eingeführten Innenkanüle (4) anliegt.

4. Trachealbeatmungsvorrichtung nach einem der Ansprüche 1 bis 3, **dadurch gekennzeichnet, dass** der Schlauchabschnitt (5) ein Teilabschnitt der Außenkanüle (2) ist, der gegenüber den hierzu distalen und proximalen Abschnitten der Außenkanüle (2) eine reduzierte Wandstärke aufweist, wobei die reduzierte Wandstärke in einem Bereich von 0,2 mm bis 0,5 mm liegt.

5. Trachealbeatmungsvorrichtung (1) nach einem der Ansprüche 1 bis 4, **dadurch gekennzeichnet, dass** der Cuffinnendruck in dem zweiten Zustand 20 hPa bis 30 hPa ist.

6. Trachealbeatmungsvorrichtung (1) nach einem der vorstehenden Ansprüche, **dadurch gekennzeichnet, dass** der Schlauchabschnitt (5) an einem radial nach außen rückspringenden Abschnitt der Innenwand der Außenkanüle (2) angeordnet ist.

7. Trachealbeatmungsvorrichtung (1) nach einem der vorstehenden Ansprüche 2 bis 6, **dadurch gekennzeichnet, dass** das maximale radiale Spiel zwischen Innenkanüle (4) und Schlauchabschnitt (5) in dem ersten Zustand zwischen 0,2 mm und 1 mm beträgt.

8. Trachealbeatmungsvorrichtung (1) nach einem der Ansprüche 1 bis 7, **dadurch gekennzeichnet, dass** der Schlauchabschnitt (5) aus einem Kunststoff mit einer Shore-Härte zwischen A50 und D50 und einer mittleren Wandstärke von 0,05 bis 0,5 mm hergestellt ist.

9. Trachealbeatmungsvorrichtung (1) nach einem der Ansprüche 2 bis 8, **dadurch gekennzeichnet, dass** in dem ersten Zustand des Schlauchabschnitts (5) die Unterbrechung ein ringförmiger Spalt (2e) mit der lichten Spaltbreite d von mindestens 0,2 mm und maximal 1 mm ist.

10. Trachealbeatmungsvorrichtung (1) nach einem der Ansprüche 1 bis 9, **dadurch gekennzeichnet, dass** die Außenkanüle (2) eine Phonationsöffnung (8), insbesondere eine Fensterung oder eine Siebung, aufweist.

## Claims

1. Tracheal ventilation device (1), in particular a tracheostomy cannula, with an outer cannula (2) and an inner cannula (4), wherein the inner cross section of the outer cannula (2) and the outer cross section of the inner cannula (4) are matched to each other in such a way that the inner cannula (4) is movable with play in the outer cannula (2), wherein the outer cannula (2) has a cuff (3) for sealing and fixing the outer cannula (2) in the trachea, wherein a radially deformable tube section (5) is provided whose ends (5a, 5b) are connected sealingly to the outer cannula (2), wherein a region of the tube section (5) between the ends (5a, 5b) of the latter is radially movable from a first state to a second state, and vice versa, and wherein, in the second state, the tube section (5) seals off the inner cannula (4) inserted into the outer cannula (2), **characterized in that** the radially movable tube section (5) lies axially between the distal and proximal ends (3a, 3b) of the cuff (3), which are connected to the outer cannula, wherein the tube section (5) is subjected to the pressure of the cuff.

2. Tracheal ventilation device (1) according to Claim 1, **characterized in that** the tube section (5) can be subjected to the pressure of the cuff by means of the fact that the tube section (5) is either
a) a partial section of the outer cannula (2) that has a reduced wall thickness in relation to the distal and proximal sections of the outer cannula (2), or
b) part of the envelope of the cuff (3), or
c) sealingly fixed with its proximal and distal ends (5a, 5b) to the inside of the outer cannula (2), and the wall of the outer cannula (2) in the region between these proximal and distal ends (5a, 5b) has an interruption or a perforation (7), such that the tube section (5) can be subjected to pressure from the outside of the outer cannula (2), wherein the perforation or interruption (6) communicates with the interior of the cuff (3).

3. Tracheal ventilation device (1) according to Claim 1 or 2, **characterized in that** the radially movable region of the tube section (5) conforms in a first state to the inner surface of the outer cannula (2) and has a radial clearance with respect to the inner cannula (4), and the radially movable region of the tube section (5) in a second state rests fully circumferentially on the outer surface of the inner cannula (4) inserted into the outer cannula (2).

4. Tracheal ventilation device according to one of Claims 1 to 3, **characterized in that** the tube section (5) is a partial section of the outer cannula (2) that has a reduced wall thickness in relation to the distal and proximal sections of the outer cannula (2), wherein the reduced wall thickness is in a range from 0.2 mm to 0.5 mm.

5. Tracheal ventilation device (1) according to one of Claims 1 to 4, **characterized in that** the cuff internal pressure in the second state is 20 hPa to 30 hPa.

6. Tracheal ventilation device (1) according to one of the preceding claims, **characterized in that** the tube section (5) is arranged on a radially outwardly protruding section of the inner wall of the outer cannula (2).

7. Tracheal ventilation device (1) according to one of Claims 2 to 6, **characterized in that** the maximum radial clearance between inner cannula (4) and tube section (5) in the first state is between 0.2 mm and 1 mm.

8. Tracheal ventilation device (1) according to one of Claims 1 to 7, **characterized in that** the tube section (5) is made of a plastic having a Shore hardness of between A50 and D50 and a mean wall thickness of 0.05 to 0.5 mm.

9. Tracheal ventilation device (1) according to one of Claims 2 to 8, **characterized in that**, in the first state of the tube section (5), the interruption is an annular gap (2e) with the clear gap width d of at least 0.2 mm and at most 1 mm.

10. Tracheal ventilation device (1) according to one of Claims 1 to 9, **characterized in that** the outer cannula (2) has a phonation opening (8), in particular a window or screen.

## Revendications

1. Dispositif de ventilation trachéale (1), notamment canule de trachéotomie, avec une canule extérieure (2) et une canule intérieure (4), la section transversale intérieure de la canule extérieure (2) et la section transversale extérieure de la canule intérieure (4) étant adaptées l'une à l'autre de telle sorte que la canule intérieure (4) peut être déplacée avec du jeu dans la canule extérieure (2), la canule extérieure (2) présentant un manchon (3) pour l'étanchéification et la fixation de la canule extérieure (2) dans la trachée, une section de tuyau (5) déformable radialement étant prévue, dont les extrémités (5a, 5b) sont reliées de manière étanche à la canule extérieure (2), une zone de la section de tuyau (5) entre ses extrémités (5a, 5b) pouvant être déplacée radialement d'un premier état à un deuxième état, et inversement, dans le deuxième état, la section de tuyau (5) étanchéifiant la canule intérieure (4) insérée dans la canule extérieure (2), **caractérisé en ce que** la section de tuyau (5) déplaçable radialement est située axialement entre les extrémités distale et proximale (3a, 3b) du manchon (3) reliées à la canule extérieure (2), la section de tuyau (5) pouvant être soumise à la pression du manchon.

2. Dispositif de ventilation trachéale (1) selon la revendication 1, **caractérisé en ce que** la section de tuyau (5) peut être soumise à la pression du manchon **en ce que** la section de tuyau (5) soit
a) est une section partielle de la canule extérieure (2) qui présente une épaisseur de paroi réduite par rapport aux sections distale et proximale de la canule extérieure (2), soit
b) fait partie de l'enveloppe du manchon (3), soit
c) est fixée de manière étanche sur le côté intérieur de la canule extérieure (2) par ses extrémités proximale et distale (5a, 5b) et la paroi de la canule extérieure (2) dans la zone entre ces extrémités proximale et distale (5a, 5b) présente une interruption ou une perforation (7), de telle sorte que la section de tuyau (5) peut être soumise à une pression depuis le côté extérieur de la canule extérieure (2), la perforation ou l'interruption (6) étant en liaison avec l'intérieur du manchon (3).

3. Dispositif de ventilation trachéale (1) selon la revendication 1 ou 2, **caractérisé en ce que**, dans un premier état, la zone déplaçable radialement de la section de tuyau (5) épouse la surface intérieure de la canule extérieure (2) et présente un jeu radial par rapport à la canule intérieure (4) et, dans un deuxième état, la zone déplaçable radialement de la section de tuyau (5) s'applique entièrement contre la surface extérieure de la canule intérieure (4) introduite dans la canule extérieure (2).

4. Dispositif de ventilation trachéale selon l'une quelconque des revendications 1 à 3, **caractérisé en ce que** la section de tuyau (5) est une section partielle de la canule extérieure (2) qui présente une épaisseur de paroi réduite par rapport aux sections distale et proximale de la canule extérieure (2), l'épaisseur de paroi réduite se situant dans une plage de 0,2 mm à 0,5 mm.

5. Dispositif de ventilation trachéale (1) selon l'une quelconque des revendications 1 à 4, **caractérisé en ce que** la pression intérieure du manchon dans le deuxième état est de 20 hPa à 30 hPa.

6. Dispositif de ventilation trachéale (1) selon l'une quelconque des revendications précédentes, **caractérisé en ce que** la section de tuyau (5) est agencée sur une section en retrait radialement vers l'extérieur de la paroi intérieure de la canule extérieure (2).

7. Dispositif de ventilation trachéale (1) selon l'une des revendications 2 à 6 précédentes, **caractérisé en ce que** le jeu radial maximal entre la canule intérieure (4) et la section de tuyau (5) dans le premier état est compris entre 0,2 mm et 1 mm.

8. Dispositif de ventilation trachéale (1) selon l'une quelconque des revendications 1 à 7, **caractérisé en ce que** la section de tuyau (5) est fabriquée en une matière plastique ayant une dureté Shore comprise entre A50 et D50 et une épaisseur de paroi moyenne de 0,05 à 0,5 mm.

9. Dispositif de ventilation trachéale (1) selon l'une quelconque des revendications 2 à 8, **caractérisé en ce que**, dans le premier état de la section de tuyau (5), l'interruption est une fente annulaire (2e) avec une largeur de fente libre d d'au moins 0,2 mm et d'au maximum 1 mm.

10. Dispositif de ventilation trachéale (1) selon l'une quelconque des revendications 1 à 9, **caractérisé en ce que** la canule extérieure (2) présente une ouverture de phonation (8), notamment une fenêtre ou un tamis.
